# EUROPEAN PATENT SPECIFICATION

(11) **EP 2 493 287 B1**
(45) Date of publication and mention of the grant of the patent: **04.10.2017**
(21) Application number: 10770638.4
(22) Date of filing: 25.10.2010
(51) Int. Cl.: A01K 11/00, A61K 9/00

(54) **Bolus system**
Bolussystem
Système de bolus

(30) Priority: 26.10.2009 EP 09174031
(43) Date of publication of application: 05.09.2012
(73) Proprietor: Nederlandse Organisatie voor toegepast- natuurwetenschappelijk onderzoek TNO, 2595 DA 's-Gravenhage (NL)
(72) Inventor: SEWALT, Karl, Everhardus, NL-3119 BT Schiedam (NL); VAN TILBURG, Mark, Wilhelmus, Theresia, NL-5691 ZC Son (NL)
(74) Representative: V.O.
(86) International application number: PCT/NL2010/050708
(87) International publication number: WO 2011/053130

(56) References cited:
- WO-A1-98/47351
- AU-B3- 649 212
- US-A1- 2004 133 131
- US-A1- 2004 155 782
- US-A1- 2006 289 640
- US-A1- 2007 008 155

## Description

### Field of the invention

The invention relates to a bolus system for identifying livestock. Such a system uses an ingestible bolus with an identification tag that can be read out from the outside after ingestion by an animal.

### Background

The identity and physiological condition of live-stock is of paramount importance to owners, buyers, and processors of livestock. It is known to visually identify livestock using brands or ear tags. It is also known to identify livestock using radio frequency identification (RFID) tags, e.g., in the form of an ear tag, an injectable tag, or an ingestible bolus which is intended to be received in a stomach, e.g. the reticulum (second pre-stomach) of a ruminant. As used in the art and in this specification, the word bolus is used for any artificial object fed to an animal. By including a sensor with the RFID tag, it is possible to collect indications of physiological characteristics, e.g., temperature etc. Apart from its use for animal identification, monitoring etc., in some cases boluses are used for internal delivery of nutrients, vitamins, medicines etc. Typically, a bolus remains in the stomach from the time that it is fed to the animal to the end of its life. To ensure this, the bolus preferably has a sufficiently large size and a weight to volume ratio that exceeds that of stomach fluids, so that it does not easily leave the stomach.

WO 98/47351 describes the use of boluses with identification tags. The document mentions the possibility of administering compounds from the bolus. Satellite carriers containing such compounds are attached to the bolus for this purpose. The document discloses an elongated bolus with a concave waste wherein the satellite carriers are anchored on the bolus. Magnetic anchoring is used, with a metallic core in the satellite carrier and a corresponding magnet in the bolus.

US 2004/133131 discloses a sensor device for monitoring pH in the stomach of an animal. To retain the sensor device in the stomach, use is made of the reticulum magnet that is sometimes fed to cattle to collect sharp metallic particles in the stomach. To attract the sensor device to this magnet, it is provided with a metallic mass, which extends along the length of the sensor device.

US 2007/008155 proposes to use a combination of an internal tag and an external tag to identify cattle. The external tag serves for visual inspection and the internal tag is used to ensure that the external tag cannot easily be replaced. The external tag is designed to communicate with the internal tag and to change state when removed from the proximity of the internal tag. Electromagnetic pulses are proposed as a means of communication.

US2006289640 discloses oral drug delivery device. The device comprises a capsule, tablet or pill. For monitoring therapy compliance, the device also comprises an RFID tag in the capsule, tablet or pill. A magnet or other electromagnetic shielding object is included to alter the antenna characteristic of the antenna of the RFID tag. When the drug delivery device disperses in the gastro-intestinal tract, the magnet or other electromagnetic shielding object separates from the antenna. Form the effect om the antenna characteristics, it is detected whether the device has dispersed.

AU649212 discloses an animal identification apparatus with a transponder in a housing. A magnetic plug is used to seal a hole in the housing, to facilitate recovery of the apparatus in order to prevent damage to equipment in the abattoir.

Known boluses, which may include RFID chips etc. for animal identification and/or monitoring, are too large to administer to newborn ruminant calves (0 - 3 days). The bolus may be stuck in and/or harm the oesophagus; newborn calves, besides, have a limited peristalsis. Known are "miniboluses" for e.g. lams and goats. Such miniboluses, however, are too small to be used for cows, as they, due to their small volume and/or low density, may be lost during ruminating, end up in another stomach or be lost via the excrements.

### Summary

One aim of the present invention is to provide a bolus system which is adaptable in size and/or density (weight), in order to be suitable to be used for newborn bovine animals (e.g. calves) as well as for mature bovine animals.

Another aim of the invention is to provide a bolus system comprising single boluses having limited dimensions, which can administered individually at subsequent points of time, thus enabling enhanced and more secure administration and installation in the (small) animal's reticulum, and enabling that, by delayed administration of additional boluses and "magnetic coagulation" within the stomach of the (growing) animal the bolus system, having then more interconnected boluses, has sufficient volume and density to prevent that the bolus system unintentionally disappears from the reticulum.

According to one aspect a bolus system is provided for installation in a stomach of an animal, comprising a central bolus having an elongate shape and comprising first and second magnetic attraction means located at mutually opposite ends of a longest extent of the elongate shape and first and second satellite boluses, each having first and second further magnetic attraction means, the first and second satellite boluses being located at the opposite ends, connected to the first and second magnetic attraction means by magnetic force, each of a first combination of the first magnetic attraction means and the first satellite magnetic attraction means, and a second combination the second magnetic attraction means and the second satellite magnetic attraction means comprising a permanent magnet. The elongate shape has different length and width measured along different directions. In the longest direction the bolus for a cow may have a length of up to 50 mm for example and a width in the shortest direction of up to 20 mm. The bolus may be at least twice as long as wide for example.

The magnetic attraction means make it possible to expand the size of the bolus system after feeding the bolus to the animal. Thus, a bolus comprising an electronically readable identification tag may be fed to the young animal when it is still small and this bolus may be expanded in the stomach of the animal, increasing its weight/volume ratio and/or its length, by feeding one or more further boluses magnetic attraction means. By locating the magnetic attraction means at the end of the longest extent of the bolus the distance between the further bolus and the identification tag in the bolus can be kept maximally large, reducing the risk of interference with information reading. Moreover, a maximum increase in length can thus be realized by feeding the further bolus.

The magnetic attraction means may be a permanent magnet or magnetizable material in the bolus at said first end. At least one of the bolus and the further bolus comprises a permanent magnet. Small neodymium magnets may be used, which may be coated by a plastic or ceramic outside layer which is inert for the biochemical compounds and processes within the animal's stomach. Unlike the prior art boluses, wherein a magnet or magnetizable material is included on the narrow side of the bolus, the magnetic attraction means are located at the end of the longest direction of the elongate shape. In the prior art boluses this location of the magnet would be undesirable because it reduces the stability of the attachment. But by using magnetic attraction means at the long end the distance between the further bolus and the identification tag in the bolus can be kept maximally large and maximum increase in length can be realized.

The bolus has first and second separate magnetic attraction means at its long ends. Thus a first and second further boluses that are fed separately from the original bolus can be attached at both ends, realizing a maximum distance and size.

In an embodiment a permanent magnet on the end of the bolus is used as magnetic attraction means, having a permanent magnetic polarization directed parallel to the length of the bolus. In this way it is promoted that the further bolus will attach in a stable way that increases the distance from the bolus to the further bolus and the length of the entire system.

When the boluses are fed to a cow separate feeding of boluses with magnetic attraction means make it possible to feed the boluses when the cow is less than three days old. In an embodiment, the second bolus may be fed to said animal after the first bolus, for example at least one day later, when the cow has grown.

Preferably at least one of the boluses administered to the animal at the first administration moment (e.g. administered to a newborn calf) includes data exchange means, e.g. an RFID tag for animal identification, and, possibly, data collection means, e.g. for animal health monitoring. Moreover, such bolus may be used as a carrier for internal delivery of nutrients, vitamins, medicines etc. But the bolus system may be applied without such carriers. After the bolus with the identification tag has been fed to the cow it may be read out electronically using wireless communication from outside the cow to identify the cow.

### Brief description of the drawing

Hereinafter, these and other objects and advantageous aspects will become apparent from a description of exemplary embodiments, with reference to the figures, wherein:
Figure 1 shows, in cross sectional view, an exemplary embodiment of the bolus system according to the invention, including a central bolus and two satellite boluses;
Figure 2 shows, in perspective view, a central bolus to be administered solely to a newborn animal;
Figure 3 shows the central bolus shown in figure 2, provided with one satellite bolus, interconnected by mutual magnetic attraction force;
Figure 4 shows the bolus system shown in figure 3, provided with a second satellite bolus, administered to the animal when growing up.

### Detailed description of exemplary embodiments.

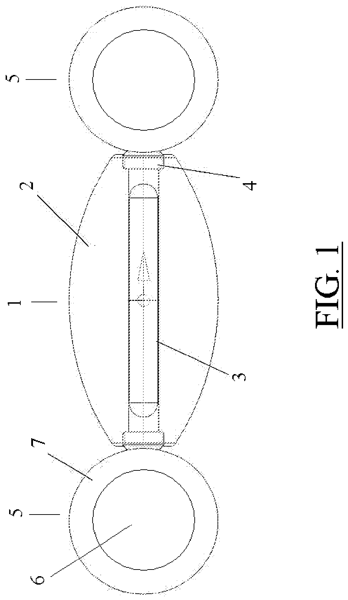

Figure 1 shows a central bolus 1, having a body 2 made from e.g. a ceramic material, which covers an RFID chip 3 or another data transmission chip, possibly including capabilities for data collection related to the animal's health etc., as e.g. suggested in US2004/0155782 and US2006/0132317. The bolus, moreover, includes two magnets 4. This central bolus 1, may be administered to a newborn calf, within 1 - 3 days after its birth, in order to be remotely identifiable.

After a certain time the calf will have become larger. To avoid that the central bolus might disappear from the reticulum of the calf, one satellite bolus 5 will be administered to the calf. Such a satellite bolus 5 includes a body 6 which is covered by e.g. a ceramic layer 7. The "magnetic attraction" body 6 is made of a permanent magnetic material like neodymium, or is made of a ferromagnetic material like iron, nickel or ferrite. After the satellite bolus 5 has arrived in the reticulum of the calf, the satellite bolus 5 will be connected, by their mutually attracting magnetic forces, to the central bolus 1 which was administered to the calf within 3 days after its birth. By this magnetic connection of the boluses 1 and 5 the total volume and density of the total bolus system 1-5 may be sufficient to prevent ejection of the bolus system from the animal's reticulum.

Additionally, a second satellite bolus 5 will be administered to the animal, which, again, is attracted by the central bolus 1, after which the volume and density of the bolus system 5-1-5, has been increased such that the whole can not leave the reticulum.

Second satellite bolus 5 may be administered when the calf has become a young cow, for example at least a plurality of days after feeding the first satellite bolus. It is also possible to feed individually all separate parts, i.e. the central bolus 1 and the satellite boluses 5, within 1 - 3 days after the calf is born, instead of waiting until the calf has grown. In this case, all separate parts may be fed one after the other, but otherwise substantially at the same time.

It is noted further that more (than two) satellite boluses may be fed to the animal. More satellite boluses 5 may be attracted one another by the magnetic field or fields generated in the central bolus 1 and possible in one or more satellite boluses.

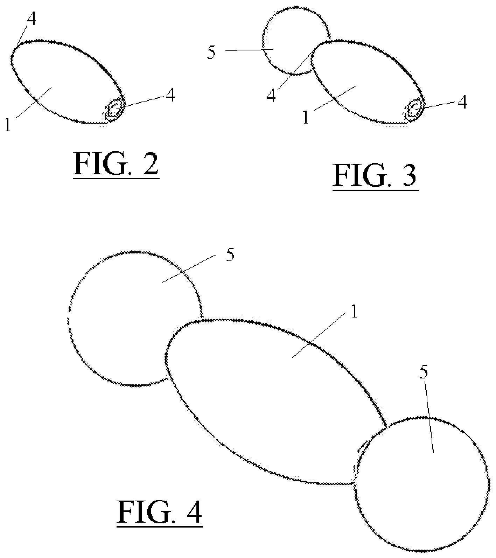

Figure 2 shows again the central bolus 1, to be administered to a newborn animal, including magnets 4 extending to its outer surface (however preferably covered by a suitable coating of cover).

Figure 3 shows the central satellite bolus 1, shown in figure 1, administered to the animal when newly born, and one satellite bolus 5 coagulated, after being administering after some months to the animal, and coagulating with the central bolus 1 by their mutual magnetic attraction force of one of the permanent magnets 4.

Figure 4 shows the bolus system, completed with a second satellite bolus 5, administered to the animal when it has become more or less mature, and thus forming a bolus system which has sufficient volume and density to prevent that it can be lost from the reticulum.

It is noted that, in particular when the satellite boluses include magnetic bodies having permanent magnetic characteristics, e.g. when they are made of neodymium, it is possible, when desired to add more (than two) satellite boluses to the bolus(es) already in the reticulum.

Central bolus 1 with RFID chip 3 is preferably substantially made of electrically non-conducting material, such as ceramic material or polymeric material. In an embodiment satellite bolus 5 and particularly its body 6 has a higher weight to volume ratio than central bolus 1. Central bolus 1 may have a relatively light weight, with a weight to volume ratio of no more than the weight to volume ratio of stomach fluids. Satellite bolus 5 may have weight to volume ratio higher than that of the stomach fluid. The relative volumes of central bolus 1 and satellite boluses 5 (or satellite bolus 5 when only one satellite bolus 5 is fed) is selected to that the weight to volume ratio of the complex of central bolus 1 and satellite bolus or boluses 5 is higher than that of the stomach fluid. As a result the complex will settle in the stomach without floating even if central bolus 1 is made of relatively light material to allow for reading of RFID chip 3.

As shown, central bolus 1 has an elongate shape, permanent magnets 4 being located at opposite ends of the bolus in its longest direction. A small distance between the RFID chip 3 and the bodies 6 of satellite boluses 5 could hamper communication with the RFID chip 3. Attachment of satellite boluses 5 at the ends of the central bolus in its longest direction makes it possible to maximize the distance between RFID chip 3 and the bodies 6 of satellite boluses 5. Furthermore, attachment of satellite boluses 5 lengthens the elongate shape. In this way the size of the system of the central bolus 1 and satellite boluses 5 in the direction of its maximum length is maximized, which reduces the risk that the bolus system will be lost.

Preferably a first and second permanent magnet 4 are used that are separate from each other and located on opposite ends of the greatest length of central bolus 1 respectively. Thus the distance between the permanent magnets 4 and RFID chip 3 is also maximal. Permanent magnets 4 may be electrically isolated from one another by using electrically isolating material in the bolus between permanents magnet 4. Substantially all of the body of central bolus outside permanent magnets 5 may be of such isolating material, to avoid interference during reading of RFID chip 3. RFID chip 3 may be located substantially midway between permanent magnets 5.

In an embodiment the direction of polarization of permanent magnets 4 is parallel to the longest direction of central bolus 1. In this way, it is promoted that satellite boluses 5 attach as shown in the figures, extending from the ends of the central bolus in its longest direction.

For the application to cows, central bolus may comprise a tube of 4 mm diameter and 32 mm length containing the RFID chip 3. The tube is of electrically non-conductive material such as glass or ceramic material. The central bolus as a whole may have a length of up to 45 mm in its longest direction, with a surface shape that corresponds to a surface of revolution around a central axis in the longest direction, with a diameter at its waist of 20 mm and 6 mm at its ends. It has been found that a maximum diameter of up to 20 mm is acceptable for feeding to a new born calf. The rounded shape with a diameter that tapers toward its ends promotes ingestion. A length of no more than 50 mm reduces the risk of damage and stagnation during ingestion. The bolus may be at least twice as long as wide for example, to realize a larger distance to the additional boluses at the ends of the elongated direction, with a width of at most 20 mm for example. Satellite boluses 5 may have a spherical shape with a diameter of up to 20 mm for example.

Although an embodiment has been shown with permanent magnets at both ends of the length of the central bolus 1, it should be appreciated that in another embodiment a magnet at only one end of the length may suffice, when only one additional bolus needs to be attached to the central bolus. This may be the case for example when the central bolus is not fed to the animal at a very early stage of growth. Although an embodiment has been shown wherein permanent magnets are used on the central bolus, it should be appreciated that instead it may suffice to use (ferromagnetic) magnetizable material, which becomes magnetic under influence of a permanent magnet on an additional bolus. Preferably both of the central bolus and the additional bolus comprise a permanent magnet or magnetizable material, at least one comprising a permanent magnet. When a central bolus with a first and second magnet at the opposite ends of its length is used, it is preferred to use a first and second permanent magnets at these ends respectively. This prevents mutual attachment of the additional boluses in the stomach separate from the central bolus.

It is noted that in US2004/0155782 and US2006/0132317 applying a permanent magnet, included in a bolus, is known as such. However, the aim of that permanent magnet, mounted in the bolus at a location separated from the transponder electronics, is to reduce the risk of hardware disease in the bolus host by attracting metal objects.

According to one aspect a bolus system is provided, intended for installing into a stomach or other organ of an animal, comprising at least one bolus (1, 5) having magnetic attraction means (4, 6) for attracting one or more other boluses (5) when present in each other's proximity. In a further embodiment the system comprises at least two boluses (1, 5) having magnetic attraction means (4, 6) for attracting one another when present in each other's proximity. At least one of the boluses may comprise at least one permanent magnet (4). At least one bolus (5) may comprise a magnetic attraction member (6). At least one of said boluses (1, 5) may include data exchange means (3) and/or data collection means. At least one of said boluses (1, 5) may be arranged for internal delivery of nutrients, vitamins, medicines etc.

## Claims

1. A bolus system for installation in a stomach of an animal, comprising a central bolus (1) having an elongate shape and comprising first and second magnetic attraction means (4) located at mutually opposite ends of a longest extent of the elongate shape and first and second satellite boluses (5), each having first and second further magnetic attraction means (6), the first and second satellite boluses (5) being located at the opposite ends, connected to the first and second satellite magnetic attraction means (6) by magnetic force, each of a first combination of the first magnetic attraction means (4) and the first satellite magnetic attraction means (6), and a second combination the second magnetic attraction means (4) and the second satellite magnetic attraction means (6) comprising a permanent magnet (4, 6).

2. A bolus system according to claim 1, wherein the permanent magnet of the first magnetic attraction means (4) has a permanent magnetic polarization directed parallel to a direction of said longest extent of the elongate shape.

3. A bolus system according to any one of the preceding claims, wherein the bolus (1) comprises an electronically readable identification tag (3).

4. A bolus system according to claim 3, wherein the bolus (1) comprises an electric isolator isolating mutually opposite first and second ends of the longest extent of the elongate shape from each other, the identification tag (3) being located in or on said electric isolator.

5. A bolus system according to any preceding claim, wherein said bolus (1) includes data collection means.

6. A method for installing a bolus system into a stomach a cow, comprising the steps of:
- feeding a first bolus (1) to the cow when the cow is at most three days old, the bolus having an elongate shape and comprising an electronic identification tag and first and second magnetic attraction means (4) located at a first and second, mutually opposite ends of a longest extent of the elongate shape respectively;
- feeding a first and second satellite bolus (5) separately form the first bolus, each of the first and second satellite bolus (5) comprising further magnetic attraction means (6) to the cow, wherein the first and second satellite bolus (5) are fed to said animal later than the first bolus (1), the first and second magnetic attraction means comprising a permanent magnet (4) and/or the further magnetic attraction means (6) of the (first and second satellite bolus (5) each comprising a permanent magnet (6).

7. A method according to claim 6, wherein the first and second satellite bolus (5) are fed to said animal at least one day after the first bolus (1).

8. A method according to claim 6, wherein the first bolus includes data collection means.

## Patentansprüche

1. Bolussystem zum Einsetzen in einen Magen eines Tieres, umfassend einen zentralen Bolus (1) mit einer länglichen Form und umfassend erste und zweite magnetische Anziehungsmittel (4) angeordnet an zueinander entgegengesetzten Enden eines längsten Ausmaßes der länglichen Form, und erste und zweite Satellitenboli (5), jeder mit ersten und zweiten weiteren Anziehungsmittel (6), welche ersten und zweiten Satellitenboli (5) an entgegengesetzten Enden angeordnet sind, verbunden mit den ersten und zweiten magnetischen Satellitenanziehungsmitteln (6) durch Magnetkraft, jede einer ersten Kombination der ersten magnetischen Anziehungsmittel (4) und der ersten magnetischen Satellitenanziehungsmittel (6) und einer zweiten Kombination der zweiten magnetischen Anziehungsmittel (4) und der zweiten magnetischen Satellitenanziehungsmittel (6) umfassend einen Dauermagneten (4, 6).

2. Bolussystem nach Anspruch 1, wobei der Dauermagnet der ersten magnetischen Anziehungsmittel (4) eine permanente magnetische Polarisation hat, die parallel zu einer Richtung des längsten Ausmaßes der länglichen Form gerichtet ist.

3. Bolussystem nach einem der vorhergehenden Ansprüche, wobei der Bolus (1) eine elektronische Identifikationsmarke (3) hat.

4. Bolussystem nach Anspruch 3, wobei der Bolus (1) einen elektrischen Isolator umfasst, der zueinander entgegengesetzte erste und zweite Enden des längsten Ausmaßes der länglichen Form voneinander isoliert, welche Identifikationsmarke (3) in oder auf dem elektrischen Isolator angeordnet ist.

5. Bolussystem nach einem der vorhergehenden Ansprüche, wobei der Bolus (1) Datenerfassungsmittel umfasst.

6. Verfahren zum Einsetzen eines Bolussystems in einen Magen einer Kuh, umfassend folgende Schritte:
- Einführen eines ersten Bolus (1) in die Kuh, wenn die Kuh höchstens drei Tage als ist, welcher Bolus eine längliche Form hat und eine elektronische Identifikationsmarke und erste und zweite magnetische Anziehungsmittel (4) umfasst, die an einem ersten und zweiten, jeweils zueinander entgegengesetzten Enden eines längsten Ausmaßes der länglichen Form angeordnet sind;
- Einführen eines ersten und zweiten Satellitenbolus (5) getrennt von dem ersten Bolus, jeder der ersten und zweiten Satellitenboli (5) umfassend weitere magnetische Anziehungsmittel (6), in die Kuh, wobei die ersten und zweiten Satellitenboli (5) später als der erste Bolus (1) in das Tier eingeführt wird, welche ersten und zweiten magnetischen Anziehungsmittel einen Dauermagneten (4) umfassen und/oder welche weiteren magnetischen Anziehungsmittel (6) des ersten und zweiten Satellitenbolus (5) jeweils einen Dauermagneten (6) umfassen.

7. Verfahren nach Anspruch 6, wobei die ersten und zweiten Satellitenboli (5) dem Tier mindestens einen Tag nach dem ersten Bolus (1) eingeführt werden.

8. Verfahren nach Anspruch 6, wobei der erste Bolus Datenerfassungsmittel umfasst.

## Revendications

1. Système de bolus destiné à une installation dans un estomac d'un animal, comprenant un bolus central (1) ayant une forme allongée, et comprenant des premiers et seconds moyens d'attraction magnétique (4) situés à des extrémités mutuellement opposées d'une étendue la plus longue de la forme allongée, et des premier et second bolus satellites (5), chacun ayant des premiers et seconds moyens d'attraction magnétique supplémentaires (6), les premier et second bolus satellites (5) étant situés aux extrémités opposées, connectés aux premiers et seconds moyens d'attraction magnétique satellites (6) par une force magnétique, chacun d'une première combinaison des premiers moyens d'attraction magnétique (4) et des premiers moyens d'attraction magnétique satellites (6), et d'une seconde combinaison des seconds moyens d'attraction magnétique (4) et des seconds moyens d'attraction magnétique satellites (6) comprenant un aimant permanent (4, 6).

2. Système de bolus selon la revendication 1, dans lequel l'aimant permanent des premiers moyens d'attraction magnétique (4) à une polarisation magnétique permanente dirigée parallèlement à une direction de ladite étendue la plus longue de la forme allongée.

3. Système de bolus selon l'une quelconque des revendications précédentes, dans lequel le bolus (1) comprend une étiquette d'identification pouvant être lue électroniquement (3).

4. Système de bolus selon la revendication 3, dans lequel le bolus (1) comprend un isolant électrique isolant des première et seconde extrémités mutuellement opposée de l'étendue la plus longue de la forme allongée l'une de l'autre, l'étiquette d'identification (3) étant située dans ou sur ledit isolant électrique.

5. Système de bolus selon l'une quelconque des revendications précédentes, dans lequel ledit bolus (1) comprend des moyens de recueil de données.

6. Méthode pour installer un système de bolus dans un estomac d'une vache, comprenant les étapes consistant à :
- fournir un premier bolus (1) à la vache lorsque la vache est vieille d'au plus trois jours, le bolus ayant une forme allongée et comprenant une étiquette d'identification électronique et des premiers et seconds moyens d'attraction magnétique (4) situés au niveau des première et seconde extrémités mutuellement opposées d'une étendue la plus longue de la forme allongée, respectivement ;
- fournir à la vache des premier et second bolus satellites (5) séparément du premier bolus, chacun des premier et second bolus satellites (5) comprenant des moyens d'attraction magnétique supplémentaires (6), dans lequel les premier et second bolus satellites (5) sont fournis audit animal après le premier bolus (1), les premiers et seconds moyens d'attraction magnétique comprenant un aimant permanent (4) et/ou les moyens d'attraction magnétique supplémentaires (6) des premiers et second bolus satellites (5) comprenant chacun un aimant permanent (6).

7. Méthode selon la revendication 6, dans lequel les premiers et second bolus satellites (5) sont fournis audit animal au moins un jour après le premier bolus (1).

8. Méthode selon la revendication 6, dans lequel le premier bolus comprend des moyens de recueil de données.
